# EUROPEAN PATENT APPLICATION

(11) **EP 2 316 492 A1**
(43) Date of publication of application: **04.05.2011**
(21) Application number: 09174620.6
(22) Date of filing: 30.10.2009
(51) Int. Cl.: A61K 49/06

(54) **Para-hydrogen labelled magnetic resonance imaging contrast agent**

(71) Applicant: Bracco Imaging S.p.A, 20134 Milano (IT)
(72) Inventor: Aime, Silvio, 10041, Torino (IT); Uggeri, Fulvio, 1-10010, Ivrea (IT)
(74) Representative: Macchetta, Francesco

(57) **Abstract**

The invention provides spin-ordered molecules and their use in MR imaging, in particular the use of para-water and spin-ordered molecules obtainable by reacting para-hydrogen enriched water with suitable reactive groups present on selected precursor.

## Description

### Technical field

The present invention refers to spin-ordered molecules and to their use in Magnetic Resonance Imaging, particularly to the use of para-water and to spin-ordered molecules obtainable by reacting para-hydrogen enriched water with suitable reactive groups present on selected precursors.

### Background

Magnetic Resonance Imaging is a powerful diagnostic modality essentially based on the visualisation of tissutal water. Therefore, the intensity of the signal in MRI is largely dependent on the local values of the longitudinal relaxation rate (R₁=1/T₁) of water protons. In MR images of human body's region the contrast arises from differences in R₁ values of different tissues. Often it has been found advantageous to administer contrast agents (CA) to improve the diagnostic potential of a MRI examination. In most cases the CA are injected into the body to show blood flow, perfusion or to assess specific physiological function. The CA are chemicals able to markedly increase proton relaxation rates of tissutal protons (mainly water). The currently available systems are grouped in the class of paramagnetic complexes (mainly Gd(III) and Mn(II) complexes) and in the class of iron-oxide particles, respectively. Increasing the proton relaxation rates will cause an increase of the intensity of the resulting signals providing contrast to those areas where the rates remain unaffected.

In principle, a signal strong enough to allow the build-up of MR images can be pursued through suitable preparation of hyperpolarised samples. This procedure has been first applied in the hyperpolarisation of monoatomic gases like ³He and ¹²⁹Xe (both spin ½ isotopes). A route commonly applied for hyperpolarisiting these nuclei consists of using a laser polarised light which is absorbed by Rb atoms which then transfer their spin-ordered to He or Xe atoms through the formation of so-called Van der Waals molecules. Once hyperpolarised, these nuclei maintain their magnetisation for a long time (if a proper container is used) and when administered to a patient they yield very good images of lungs and of the "aerial tree". Despite the several attempts it has not yet been found an efficient modality to exploit the potential of ³He and ¹²⁹Xe in other body's districts. A route to circumvent this problem has been tackled by hyperpolarising ¹³C-nuclei (endowed with long T₁) via the NCP procedure which consists of transferring polarisation from electron transitions. In this procedure, the ¹³C- containing substrate is first mixed with a stable organic radical, then the mixture is brought to very low temperature (e.g. from 1 to few °K) where, by applying a suitable epr irradiating frequency, polarisation transfer occurs. The separation of ¹³C- containing agent and the organic radical has to occur very efficiently otherwise most (or the whole) polarisation effect is lost. The agent containing the hyperpolarised ¹³C nuclei is then administered as done for the currently available CAs and the obtained ¹³C-image optionally fused with the proton image of the same subject. Overall, the procedure is rather cumbersome either in terms of time and technical difficulties. Another procedure has been recently devised aimed at the preparation of hyperpolarized ¹³C-containing substrates through the reaction of unsaturated derivatives with para-H₂ (WO 99/24080). In fact when the two hydrogens of the p-H₂ molecule are transferred to the unsaturated derivative, their nuclei maintain the relative orientation they had in the hydrogen molecule. Therefore, of the four energy states expected for an AX spin-system, the |αβ> and |βα> states are largely overpopulated in respect to |αα> and |ββ> states. The n.m.r. transitions are then dramatically altered. As far as the MRI application is concerned, it has been found more convenient in place of the detection of ¹H-images to take advantage of the intramolecular magnetisation transfer and to detect ¹³C-images of resonance (e.g of keto or carboxylate groups and the like, etc.) endowed with long T₁ values. The main limitation of this approach is related to the type of ¹³C-substrates that can add a molecule of p-H₂. In fact, only derivatives containing double or triple bond can be considered. Moreover the hydrogenation step requires a catalyst which often requires non-aqueous solvent thus implying an additional separation step before having the agent ready for the administration.

### Description of the invention

The present invention provides the MRI use of para-water or of a compound obtainable by reacting p-water with a precursor comprising a non hydrogen non-zero nuclear spin atom and at least one group reaction with water to give compound having at least two p-hydrogen atoms in a position adjacent to said non hydrogen non-zero nuclear spin atom, for the preparation of a diagnostic composition for use in non-proton MRI imaging.

The invention also refers to a compound obtainable by reacting p-water with a precursor comprising a non hydrogen non-zero nuclear spin atom and at least one group reacting with water to give a compound having two para-hydrogen atoms in a position adjacent to said non hydrogen non-zero nuclear spin atom.

According to a further embodiment, the invention also provides a method of magnetic resonance analysis of a sample, comprising:
a. reacting para-water with a precursor comprising a non hydrogen non-zero nuclear spin atom and at least one group reacting with water to give a compound having two-para-hydrogen atoms in a position adjacent to said non hydrogen non-zero nuclear spin atom;
b. contacting the compound obtained in a. with the sample;
c. subjecting the sample to magnetic resonance analysis detecting the signal from of said non hydrogen non-zero nuclear spin atom.

### Detailed description of the invention

It is known that ordinary water is a mixture of para and ortho-water. In analogy to para-H₂, para-water too is NMR silent. Para-water might be obtained either by reaction of p-H₂ with oxygen or by separating it from ortho-water in ordinary water. In the latter case, gas chromatography on charcoal or other inorganic support may be used.

At the liquid state, the loss of spin-order of p-water takes few minutes whereas at the solid state the time involved is order of months. Such a transformation can be easily detected by the MRI technique as the proton signal from phantoms containing para-water steadily increases as the content of para-water decreases until the thermodynamic equilibrium between ortho- and para-isomers is reached. Analogously when an aliquot of para-water is injected to a living organism, one first observe a loss of signal in the corresponding image followed by the progressive return to the steady-state condition if the images are taken at longer times.

The use of para-water in diagnostic protocols is of high relevance as it can replace any PET procedure based on the use of H₂¹⁵O with the advantage of avoiding the use of radioactive material and the attainment of much higher spatial resolution in the resulting MR images. The MRI expert will find very profitable the use of para-water in a number of protocols aimed at assessing flow and organ perfusion. This will allow the set-up of novel procedure for instance in the field of the evaluation of drugs acting on the nervous system as well as on the assessment of apoptotic processes in cancer therapies.

As the prototropic exchange is catalysed by H⁺ ions one may envisage a simple use of para-water imaging to create pH-maps in a given body's region. pH-sensitive images are of interest in cancer diagnosis as it is known that the extracellular region of many tumors displays a pH value which is ca.0.6 pH unit lower than the physiological pH value. The presence of paramagnetic or ferromagnetic substances causes a dramatic reduction of the time associated to the loss of spin-order in para-water. This may be exploited to design experiments in which a region of interest (for instance, the surface of thrombi or of an atherosclerotic plaque or the neo-formed endothelium, etc. ) is first targeted with suitably functionalised para- or ferro-magnetic probes. Already at the first passage of the para-water bolus in the region containing the magnetically active compounds, a signal increase is detected in the corresponding image in respect to the analogous image taken in para-water without the use of the pre-targeting agent.

The derivatives of the invention are obtained by reacting para-water with a suitable substrate, for instance according to the following reaction scheme:

S+p-H₂O→pS(OH)(H)

The product p-S(OH)(H) has the analogous properties of the products obtained from the reactions of unsaturated substrates with para-H₂. In this context, a number of advantages is involved in the use of para-water as an alternative to para-H₂. First of all, the possibility of using the same para-water as solvent is a clear advantage. Several reactions may be used for the addition of the para-water molecule to the substrate S. Examples of suitable precursors are the following :
1. S = activated heterocyclic systems, e.g. epoxides 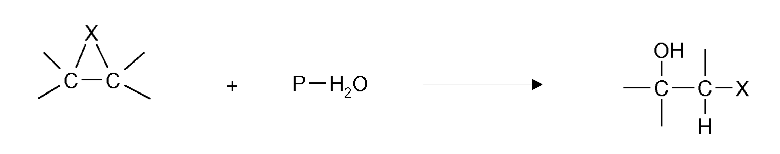
2. S = activated di-imines 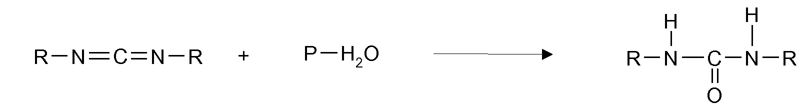
3. S = ketenes 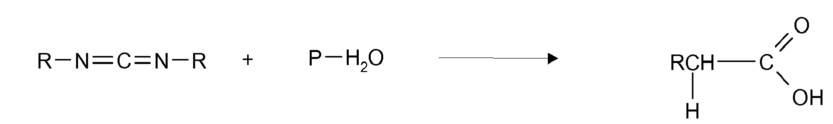
4. S = isocyanates

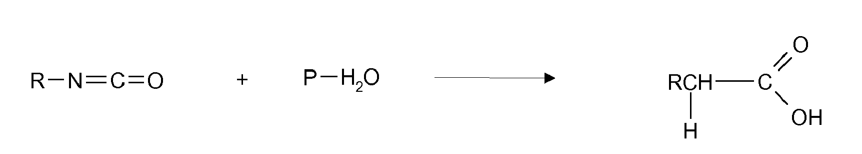

The groups R, which may be the same or different, may be selected within a large groups of compounds, provided the following characteristics for the final compound are met:
- the molecular weight is lower than about 1000 preferably lower than 500 daltons;
- the solubility characteristics are compatible with the intended use; in case of in vivo administration the R groups may be substituted by moieties increasing the water solubility, such as carboxy, amino and/or hydroxy groups;
- the two p-hydrogen atoms are adjacent to the non-zero nuclear spin atom; it may either be directly linked to the non hydrogen non-zero nuclear spin atom or be separated 1 to 3 bonds therefrom, so that the coupling constant between the p-hydrogen and the non-zero nuclear spin atoms is from about 10 to about 100 Hz;
- the R groups do not contain moieties which may interfere with the reaction with para-water, if present, said moieties should be suitably protected prior to the reaction with water and de-protected in suitable conditions before the intended use, if necessary.

The non hydrogen non-zero nuclear spin atom is preferably selected from ¹³C, ¹⁵N,²⁹Si, ³¹P, ¹⁹F, preferably ¹³C or ¹⁵N.

Examples of suitable R groups include C₁-C₆ alkyl groups optionally unsaturated and optionally substituted by one to four substituents, which are the same or different, selected from C₁-C₆-alkoxy,oxo, hydroxy, amino, carboxy, C₁-C₆ alkoxycarbonyl groups; phenyl optionally substituted by one to four substituents, which are the same or different, selected from C₁-C₆-alkoxy, hydroxy, amino, carboxy, C₁-C₆ alkoxycarbonyl, cyano, halogen, nitro, C₁-C₃-acyl, solfonium or phosphonium groups; phenyl-C₁-C₂-alkyl, the phenyl group being possibly substituted by the same substituents specified above.

The preparation of said compounds enriched in the desired isotope at the given site may be carried out by usual, well-known synthetic methods starting from appropriate isotope-labelled reagents and intermediates.

The most advantageous way to exploit the para-hydrogenated molecules obtained according to the invention consists of recording the MR images of a ¹³C nucleus endowed with a relaxation time of at least 2 seconds, preferably more than 8 seconds, most preferably more than 20 seconds. Such carbons are preferentially represented by carbonylic carbons, alcoholic carbons, and preferentially carboxylate carbons. One may preferentially use a substrate containing a ¹³C-enriched position such that one may deal with a labelled carbon at the site responsible for providing the signal for the MR image. Moreover, in order to increase the relaxation time of the resonance of interest for the MR image, it may be better to use perdeuterated S substrates. Clearly the use of a ¹³C-resonance as the source of the signal for the MR image in a para-hydrogenated molecule is related to the intermolecular transfer of spin-polarisation from the p-hydrogenated moiety to a given ¹³C-transition. This is known to occur as it has been reported by Bargon et al. (J.Am.Chem.Soc., 117, 2927 (1995) and exploited in MRI as reported by Golman et al. (Magn.Res. Med., 46, 1 (2001)). These authors used the product of the reaction between acetylendicarboxylic methyl ester and para-hydrogen. The present invention widen the range of molecules available for recording ¹³C-images (or other nuclei) through the exploitation of para-hydrogen effects.

## Claims

1. The use of para-water or of a compound having two para-hydrogens deriving from the reaction comprising a suitable precursor with para-water for the preparation of a contrast agent for the magnetic resonance analysis of a sample.

2. The use according to claim 1 of a compound obtainable by reacting p-water with a precursor comprising a non hydrogen non-zero nuclear spin atom and at least one group reacting with water to give a compound having two p-hydrogen atoms in a position adjacent to said non hydrogen non-zero nuclear spin atom, for the preparation of a diagnostic composition for use in non-proton MR imaging.

3. The use according to claim 2 wherein said non hydrogen non-zero nuclear spin atom is selected from ¹³C, ¹⁵N, ²⁹Si, ³¹P, ¹⁹F.

4. The use according to claim 3 wherein said non hydrogen non-zero nuclear spin atom is selected from ¹³C and ¹⁵N.

5. The use according to any one of claims 1-4, wherein said group reacting with water is selected from epoxy, ketene, di-imino and isocyanate groups.

6. The use according to any one of claims 1-5, wherein said p-hydrogen atom are either directly linked to the non hydrogen non-zero nuclear spin atom or are distant 1 to 3 bonds therefrom.

7. A compound obtainable by reacting p-water with a precursor comprising a non hydrogen non-zero nuclear spin atom and at least one group reacting with water to give a compound having two p-hydrogen atoms in a position adjacent to said non hydrogen non-zero nuclear spin atom.

8. A compound according to claim 7 wherein said non hydrogen non-zero nuclear spin atoms is selected from an isotope selected from ¹³C, ¹⁵N, ²⁹Si, ³¹P, ¹⁹F.

9. A compound according to claim 7 or 8 wherein said group reacting with water is selected from epoxy, ketene, di-imino and isocyanate groups.

10. A method of magnetic resonance analysis of a sample, comprising:
a) reacting para-water with a precursor comprising a non hydrogen non-zero nuclear spin atom and at least one group reacting with water to give a compound having two p-hydrogen atoms in a position adjacent to said non hydrogen non-zero nuclear spin atom;
b) contacting the compound obtained in a. with the sample;
c) subjecting the sample to magnetic resonance analysis detecting the signal from said non hydrogen non-zero nuclear spin atom.

11. A method according to claim 10 wherein said non hydrogen non-zero nuclear spin atom is selected from ¹³C, ¹⁵N, ²⁹Si, ³¹P, ¹⁹F.
